Europäisches Patentamt

European Patent Office

Office européen des brevets

(19)

(11) Veröffentlichungsnummer: **0 196 146**
**A2**

(12) # EUROPÄISCHE PATENTANMELDUNG

(21) Anmeldenummer: **86200565.9**

(51) Int. Cl.⁴: **C 12 P 21/02**

(22) Anmeldetag: **19.03.86**

(30) Priorität: **27.03.85 DE 3511011**

(71) Anmelder: **HOECHST AKTIENGESELLSCHAFT,
Postfach 80 03 20, D-6230 Frankfurt am Main 80 (DE)**

(43) Veröffentlichungstag der Anmeldung: **01.10.86
Patentblatt 86/40**

(84) Benannte Vertragsstaaten: **AT BE CH DE FR GB IT LI LU
NL SE**

(72) Erfinder: **Obermeier, Rainer, Dr., Langenhainer Weg 14,
D-6234 Hattersheim am Main (DE)**

(54) **Verfahren zur Isolierung und Reinigung von alpha-Interferonen.**

(57) Die Erfindung betrifft ein Verfahren zur Isolierung und Reinigung von α-Interferonen durch a) Lösen des interferonhaltigen Rohprodukts in Guanidin · HCI-Lösung und Ausfällen durch Verdünnen mit Wasser und/oder b) durch Chromatographie über eine HIC-Säule unter renaturierenden Bedingungen.

EP 0 196 146 A2

## Verfahren zur Isolierung und Reinigung von α-Interferonen

Bereits 1956 entdeckten Isaacs und Lindemann Interferon als einen indirekten Hemmstoff der intracellulären Virusvermehrung. Inzwischen wurde eine große Anzahl verschiedener Interferontypen natürlicher Herkunft identifiziert, die vor allem durch die unterschiedlichen Herkunftszellen charkterisiert werden:

α-Interferone aus Leukozyten

ß-Interferone aus Fibroblasten

γ-Interferone aus Lymphozyten.

Die durch entsprechende Stimuli in den jeweiligen Zellen induzierten Interferone gehören chemisch zur Gruppe der Glykoproteine, sind z.T. säurestabil (pH 2 -3) und entfalten ihre volle antivirale Wirkung von $10^8$ - $10^9$ IE/mg spezies-spezifisch. Die Primärstruktur einer Reihe von α-, ß- und γ-Interferonen, die aus 146 - 166 Aminosäuren aufgebaut sind ist aufgeklärt. Daneben sind nicht in der Natur vorkommende Interferone bekannt geworden, deren Struktur auf gentechnologischem Wege entweder verkürzt, oder gegenüber den natürlichen Interferonen in der Aminosäuresequenz abgeändert wurden.

Jüngste Arbeiten auf dem Gebiet der Gentechnologie, bei denen Interferon-Gene in E. coli zur Expression gebracht wurden, haben gezeigt, daß die z.T. in den natürlichen Interferonen nachgewiesenen Glycosyl- bzw. Polysaccharid-Seitenketten keinen Einfluß auf die biologische Aktivität der Proteine zu haben scheinen. Darüber hinaus kann mit Antikörpern, die mit dem natürlichen Glykoprotein erzeugt wurden, eine vergleichbare Bindung an nicht glykosylierte Interferone aus E. coli gemessen werden, was auf den überwiegenden Einfluß der Proteinstruktur des Interferons als Antigendeterminate hindeutet.

Wegen ihrer therapeutischen Potenz werden die Interferone im großtechnischen Maßstab entweder durch Ernten von Kulturmedien entsprechender Zellkulturen, oder neuerdings durch Fermentation von E. coli-Stämmen, in die auf gentechnologischem Wege ein Interferon-codierender DNA-Vektor cloniert wurde produziert. Zur Isolierung und Reinigung der gewonnenen Rohinterferon-Präparate (durchschnittliche Aktivität $10^3$ - $10^4$ IE/mg Protein) werden im allgemeinen Verfahren der Affinitäts- und Adsorptionschromatographie verwendet. Diese nützen die spezielle Fähigkeit des Interferons an immobilisierte hydrophobe Liganden, Metallionen, Thiole, organische Quecksilberverbindungen, Polynukleotide, Controlled Pore Glass, sowie an Antikörer mit hoher Spezifität zu binden. Trotz der Vielfalt von Reinigungsmethoden bleiben Ausbeuten und Reinheit der damit erhaltenen Interferone jedoch unbefriedigend, wie die Unsicherheit über die Ursache der Nebenwirkungen bei der klinischen Prüfung der Interferone am Menschen zeigt.

Besonders die Denaturierungsneigung der Interferone bereitet erhebliche Schwierigkeiten und ergibt werden der Bildung von polymeren unlöslichen Aggregaten zum Teil hohe Verlust bei der Reinigung und Isolierung von Interferon. Neue, effektive Isolierungsverfahren sind deshalb notwendig.

Diese Aufgabe wird erfindungsgemäß gelöst durch ein Verfahren zur Isolierung und Reinigung von plasmidogenen α-Interferonen aus gentechnologisch veränderten Bakterienkulturen und α-Interferonen aus Kulturüberständen von induzierten Sägetierzellen, das dadurch gekennzeichnet ist, daß man das α-Interfon aus ensprechenden vorzugsweise gefriergetrockneten Rohprodukten mit einem Gehalt an denaturiertem α-Interferon

a) unter denaturierenden Bedingungen in vorzugsweise hochkonzentrierter wäßriger Guanidin-Hydrochlorid-Lösung löst und dann nach Abtrennung von Verunreinigungen vorzugsweise durch langsames stufenweises Verdünnen mit Wasser angereichertes α-Interferon in schwerlöslicher, oligomerer, denaturierter Form ausfällt und/oder aus,

b) auf ein HIC-Säure (Säule zur hydrophoben Interaktions-Chromatographie) aufbringt und dann das adsorbierte α-Interferon mit einer wäßrigen vorzugsweise nichtionischen Tensidlösung oder einer wäßrigen Salzlösung mit einem Salzgehalt unter 0,1 M unter renaturierenden Bedingungen eluiert.

Die Verfahrensschritte (a) und (b) können sowohl für sich allein als auch aufeinanderfolgend durchgeführt werden.

Bei aufeinanderfolgender Durchführung der Schritte (a) und (b) hat das erfindungsgemäße Verfahren gegenüber den bekannten Verfahren den Vorteil, die Eigenschaften von denaturierten Interferon zur Anreicherung und Reinigung zu nutzen und erst im letzten Schritt der chromatographischen Reinigung die Renaturierung zum nativen Interferon zu erlauben. Damit wird das bei allen anderen Verfahren unerläßliche und wegen der immer wieder notwendigen Abtrennung von denaturiertem Interferon verlustreiche Arbeiten mit nativem Interferon umgangen. Viemehr wird dieses erst auf der letzten Stufe der Reinigung verfahrensinhärent erzeugt.

Verfahrensschritt (a) wird vorzugsweise so durchgeführt, daß man entsprechende interferonhaltige Rohmaterialien, in denen das Interferon weitgehend denaturiert vorliegt unter bekannten denaturierenden Bedingungen in 6 - 8 M Guanidin · HCl gegebenenfalls unter Zugabe eines der üblichen Redaktionsmittel wie ß-Merkaptoethanol, Dithiothreitol oder Glutothion (C. Ghélis, J. Yon in Protein Folding, Academic Press, N.Y. London, ed. B. Horecker, N.O. Kaplan, J. Marmur, H.A. Scheraga, 1982, Seite 254 ff.) löst und durch vorsichtiges Verdünnen mit Wasser stufenweise Verunreinigungen ausfällt. Dabei muß der denaturierte Zustand des Interferons erhalten bleiben, so daß es in einer späten Verdünnungsstufe bei 0,5 bis 2 M Guanidin • HCl-Gehalt präzipitiert und als denaturiertes Interferon in angereicherter Form isoliert werden kann.

Dieses Material, das gewöhnlich einen Interferongehalt von mehr als 10 % aufweist, kann mittels Verfahrensschritt (b) weiter gereinigt werden.

Vorzugsweise löst man es dazu unter denaturierenden Bedingungen in ca. 6 M Guanidin • HCl und bringt es nach schneller Verdünnung mit Wasser auf einen Gehalt von ca. 1 - 3 M Guanidin • HCl gegebenenfalls unter Zusatz eines der oben beschriebenen Reduktionsmittel auf eine HIC-Säule auf.

Geeignete Gele für die HIC sind n-Butyl-TSK (Butyl-[R]Toyopearl 650 M). Diese für die HIC verwendeten Gele haben die Eigenschaft, Interferon bei hohen Salzkonzentrationen zu absorbieren. Die Desorption erfolgt durch Verringerung der Salzkonzentrationen durch Anwendung eines Wasser-Gradienten der 0,5 % [R]Tween 20 enthält, also bei renaturierenden Bedingungen. Im Unterschied zur üblichen HIC (Masateru Shin, Naoko Sakihama, Reiko Oshino and Hiroo Sasaki Anal. Biochem. 138, 259 - 261, 1984) wurden gefunden, daß die Adsorption von Interferon auch in denaturierter Form aus Guanidin • Cl erfolgt, während die üblichen Bedingungen nicht-denaturierende Salzlösungen wie Ammoniumsulfat-Lösungen erfordern, die native globuläre Struktur der Proteine stabilisieren.

Anwendungsbeispiel:

1. 100 g eines lyophilisierten Zellaufschlußes eines Interferon-produzierenden E. coli-Stammes werden in 2 l 8 M Guanidin • HCl-Lösung bei pH 7 gelöst und zur Klärung der Lösung zentrifugiert. Die Lösung des Rohmaterials wird in 6 l Wasser eingerührt und der entstehende Niederschlag über Nacht im Kühlraum sedimentieren lassen. Das Sediment wird durch Zentrifugation isoliert und feucht erneut in 8 M Guanidin · HCl (250 ml) gelöst. In die Lösung werden durchschnittlich in fünf aufeinanderolgenden Schritten jeweils 125 ml Wasser eingerührt und die entstehenden Niederschläge in jedem Schritt abzentrifugiert. Bei weiterer Zugabe von 500 ml Wasser entsteht beim Nachrühren ein feiner gelartiger Niederschlag, der gemäß radioimmunologischer Messung etwa 80 % des gesamten Interferons des Ausgangsmaterials enthält. Das feuchte abzentrifugierte Präzipitat wird bei -18°C gelagert.

2. HI-Chromatographie: 0,2 g des denaturierten Rohinterferons werden in 6 ml 6 M Guanidin · HCl-Lösung gelöst, mit 6 ml Wasser verdünnt und auf eine mit $^R$Fraktogel-TSK-Butyl-650 gefüllte Chromatographiesäule (2,5 x 30 cm) aufgetragen, die mit 3 ml Guanidin · HCl äquilibriert ist. Die Entwicklung der Chromatographie wird mit einem Durchfluß UV-Spektrophotometer bei 280 nm überwacht. Die Säule wird solange mit 3 M Guanidin · HCl entwickelt, bis ein anfänglicher Peak eluiert ist. Danach wird mit Wasser, das 0,5 % $^R$Tween-20 enthält gewaschen. Fraktionen von etwa 2 ml werden aufgefangen. Die Flußgeschwindigkeit liegt bei 1,5 ml/min. Die Hauptmenge des Interferons erscheint nach etwa 35 Fraktionen. Die vereinigten Fraktionen können mit Hilfe der Ultrafiltration bis zum gewünschten Interferongehalt aufkonzentriert und auf einen entsprechenden Puffer eingestellt werden. Das so gewonnene α-Interferon ist gemäß der SDS-Gelektrophorese (ohne Reduktionsmittel) monomer und besitzt eine spezifische Aktivität von $3,7 \cdot 10^8$ IF-Einheiten pro mg Protein, was einer Reinheit von etwa 74 % entspricht, bezogen auf einen externen IF-Standard von $5 \cdot 10^8$

IFE/mg Protein. Der Proteingehalt wird mit Hilfe der Lowry-Methode gemessen. Die Interferonaktivität wird als biologische Aktivität über den cytopathischen Effekt von Vesicular Stomatitis Virus gegen Rindernierenzellen (MDBK) und radioimmunologisch bestimmt. Die Werte der Aminosäureanalyse stimmen weitgehend mit den theoretischen Werten überein.

0196146

Patentansprüche:

1. Verfahren zur Isolierung und Reinigung von plasmidogenen α-Interferonen aus gentechnologisch veränderten Bakterienkulturen und α-Interferonen aus Kulturüberständen von induzierten Säugetierzellen, dadurch gekennzeichnet, daß man das α-Interferon aus entsprechenden Rohprodukten mit einem Gehalt an denaturiertem α-Interferon

   a) unter denaturierenden Bedingungen in wäßriger Guanidin-Hydrochlorid-Lösung löst und dann durch Verdünnen mit Wasser angereichertes α-Interferon in schwerlöslicher, oligomerer, denaturierter Form ausfällt und/oder

   b) auf ein HIC-Säule aufbringt und dann das adsorbierte α-Interferon mit einer wäßrigen Tensidlösung oder einer wäßrigen Salzlösung mit einem Salzgehalt unter 0,1 M unter renaturierenden Bedingungen eluiert.

2. Verfahren gemäß Anspruch 1, dadurch gekennzeichnet, daß man die Verfahrensschritte (a) und (b) nacheinander durchführt.

3. Verfahren gemäß Anspruch 1, dadurch gekennzeichnet, daß man entweder den Verfahrensschritt (a) oder den Verfahrensschritt (b) durchführt.

4. Verfahren gemäß einem der Ansprüche 1 bis 3, dadurch gekennzeichnet, daß α-Interferone aus gentechnologisch veränderten Bakterienkulturen isoliert und reinigt.

5. Verfahren gemäß Anspruch 4, dadurch gekennzeichnet, daß man α-Hybridinterferone isoliert und reinigt.

6. Verfahren gemäß einem der Ansprüche 1 bis 5, dadurch gekennzeichnet, daß beim Verfahrensschritt (a) das Verdünnen mit Wasser stufenweise erfolgt.

7. Verfahren gemäß einem der Ansprüche 1 bis 5, dadurch gekennzeichnet, das bei Verfahrensschritt (b) mittels einer wäßrigen Lösung eines nichtionischen Tensids eluiert wird.